# EUROPEAN PATENT APPLICATION

(11) **EP 4 299 580 A1**
(43) Date of publication of application: **03.01.2024**
(21) Application number: 22759922.2
(22) Date of filing: 25.01.2022
(51) Int. Cl.: C07K 1/14, C07K 1/12, C07K 1/36, C07K 14/435

(54) **HIGH-YIELD PURIFICATION METHOD FOR TARGET PROTEIN**

(30) Priority: 26.02.2021 KR 20210026617
(71) Applicant: SK Bioscience Co., Ltd., Bundang-gu, Seongnam-si, Gyeonggi-do, 13494 (KR)
(72) Inventor: KIM, Hak, Seongnam-si, Gyeonggi-do 13494 (KR); KWON, Taewoo, Seongnam-si, Gyeonggi-do 13494 (KR); SEO, Ki-Weon, Seongnam-si, Gyeonggi-do 13494 (KR)
(74) Representative: Dehns
(86) International application number: PCT/KR2022/001307
(87) International publication number: WO 2022/182003

(57) **Abstract**

The present invention relates to a method for purifying a target protein in high yield. More specifically, the present invention relates to a method for purifying a membrane protein in high yield. The purification method according to the present invention optimizes crushing and elution conditions during processes of separation and purification of membrane proteins, and when using same to purify membrane proteins, the membrane proteins can be purified in a higher yield that is not less than 100 times than that when membrane proteins are purified by using a conventional homogenizer or sonic pulverization. In addition, when membrane proteins are purified by using the purification method of the present invention, nuclei, peroxisomes, and lysosomes are removed, thereby reducing DNA contamination and protein damage by proteases. Therefore, the present invention can be usefully used to purify membrane proteins.

## Description

### Technical Field

The present invention relates to a method for purifying target proteins in high yield. More specifically, the present invention relates to a method for purifying membrane proteins in high yield.

### Background Art

Membrane proteins are important proteins that transmit signals inside and outside cells, and are associated with various diseases. Specifically, the membrane proteins generate differences in ion concentrations inside and outside the cells, or are used for ATP synthesis, and are involved in the generation and transmission of electrical signals in nerve cells or muscle cells. In particular, the membrane proteins are a main target of active pharmaceutical ingredients and are also a target of therapeutic monoclonal antibodies that are recently being developed.

Up to date, a large number of membrane proteins are still unknown, and research is needed to clarify the characteristics and functions of the membrane proteins. However, since the membrane proteins have higher hydrophobicity than proteins present in the cytoplasm, and exist in the lipid bilayer, the extraction and purification are not easy, and the progress of related research is slow. In the membrane protein-related research, there is a limitation in that the amount of the membrane proteins themselves present in the cell membrane is very small, and the cell membrane in which the membrane proteins are present has a large amount of fat and sugar, so that it is difficult to purify the membrane proteins.

Currently, a method for extracting membrane proteins using detergent has been developed as a method for separating and purifying membrane proteins by Molloy *et al*., and is characterized by a three-step extraction method by using urea, thiourea, CHAPS, and the like. According to a method by Molloy *et al*., cells or tissue is crushed, the supernatant is removed through the centrifugation, precipitates including membrane proteins are collected, and membrane proteins bound to lipid layers or cell walls are extracted by using detergent. Detergents mainly used in this method include Triton-X100, Nonidet P-40 (NP-40), 4-octylbenzoyl amidosulfobetine, ASB14, and the like. In addition, another method for separating and purifying the membrane is to centrifuge a sample. In this method, cells are crushed and centrifuged to separate a membrane fraction. The disadvantage of this method is that an additional verification process is required and it takes a long time.

In addition, known are labeling and chromatography methods, a method for selectively concentrating proteins by ultrafiltration having different characteristics at pH, a method for separating phosphate peptides from non-phosphate peptides by using resins and concentrating the separated phosphate peptides, and a method for selectively concentrating membrane proteins by means of phase partitioning using glass beads (M. Walid Qoronfleh et al., J Biomed Biotechnol., 2003 Oct 29; 2003(4): 249-255). However, the membrane protein separation and purification technologies of the related arts have not yet provided a satisfactory yield. Therefore, there is a need for research and development on a technology for separating and purifying membrane proteins with high efficiency.

### [Prior Art Document]

### [Non-Patent Document]

(Non-Patent Document 1) M. Walid Qoronfleh et al., J Biomed Biotechnol., 2003 Oct 29; 2003(4): 249-255.

### Disclosure of the Invention

### Technical Problem

Accordingly, the present inventors have studied to develop a technology for separating and purifying membrane proteins with high efficiency, and as a result, established optimal disruption and elution conditions, and found that membrane proteins can be obtained in high yield when the membrane proteins are separated and purified using the above conditions, thereby completing the present invention.

### Solution to Problem

In order to achieve the above object, an aspect of the present invention provides a method for purifying target proteins, the method including the steps of: i) disrupting cells using a sonicator, a homogenizer, or a microfluidizer; ii) obtaining cell membrane fractions; iii) disrupting the cell membrane fractions using the sonicator, the homogenizer, or the microfluidizer; and iv) separating the target proteins.

### Advantageous Effects of Invention

The purification method according to the present invention has optimized disruption and elution conditions during processes of separating and purifying membrane proteins, and when the purification method of the present invention is used to purify the membrane proteins, the membrane proteins can be purified at a yield which is at least 100 times higher than the case where membrane proteins are purified by using a conventional homogenizer or sonication. Also, when the membrane proteins are purified by using the purification method of the present invention, nuclei, peroxisomes and lysosomes are removed, and thus DNA contamination and protein damage by proteases can be reduced. Therefore, the present invention can be effectively used to purify membrane proteins.

### Brief Description of Drawings

FIG. 1 is a schematic diagram illustrating a step of optimizing the disruption and elution conditions in a process of separating and purifying membrane proteins.
FIG. 2 shows the results of disruption and elution experiments by using a homogenizer.
FIG. 3 shows the results of disruption and elution experiments by using a sonicator.
FIG. 4 shows the results of disruption and elution experiments by using a microfluidizer.
FIG. 5 shows comparison of the results of disruption and elution experiments by using a homogenizer, a sonicator, and a microfluidizer.

### Best Mode for Carrying out the Invention

Hereinafter, the present invention will be described in more detail.

An aspect of the present invention provides a method for purifying target proteins, the method including the steps of: i) disrupting cells using a sonicator, a homogenizer, or a microfluidizer; ii) obtaining cell membrane fractions; iii) disrupting the cell membrane fractions using the sonicator, the homogenizer, or the microfluidizer; and iv) separating the target proteins.

The homogenizer, the sonicator, or the microfluidizer may physically disrupt the membrane fraction so that chemical extraction may proceed more efficiently. In the case of general chemical extraction, a small amount of cell pellets or membrane fractions are disrupted by pipetting or the like, and then cultured at a predetermined time and temperature while stirring, shaking, rocking, and the like, but when proteins are extracted from a large amount of cell pellets or membrane fractions, it is difficult to disrupt them by using pipetting alone and extraction efficiency is reduced. In this case, the application of the homogenizer, sonicator, or microfluidizer homogenizes disruption to a very small size so that the extraction efficiency increases and cultivation time decreases, thereby obtaining target proteins more efficiently.

### Method for Purifying Target Proteins by Using Microfluidizer

In step i), the microfluidizer may be repeatedly performed once to seven times at a pressure of 3,500 psi to 6,000 psi. Specifically, in step i), the microfluidizer may be performed at a pressure of 3,500 psi to 6,000 psi, 3,800 psi to 5,700 psi, 4,100 psi to 5,400 psi, or 4,400 psi to 5,100 psi. In addition, in step i), the microfluidizer may be repeatedly performed once to seven times, twice to six times, or three to five times. In one embodiment of the present invention, in step i), the microfluidizer was repeatedly performed three to five times at a pressure of 5,000 psi.

In step i), a lysis buffer that does not contain a surfactant may be used during cell disruption. When the surfactant is contained in the lysis buffer during the cell disruption in step i), the nuclear membrane bursts and DNA is leaked, which makes it difficult to separate pure target proteins. In addition, the target proteins may be damaged by proteases leaked due to the burst of membranes of endosomes, lysosomes, or the like.

Specifically, in step i), the lysis buffer that does not contain the surfactant may be used, which may include Tris, EDTA, NaCl, and a protease inhibitor. In one embodiment of the present invention, a solution including 25 mM Tris, 1 mM EDTA, 100 mM NaCl and a protease inhibitor and having pH 8.5 was used as a lysis buffer without containing a surfactant during the cell disruption.

In step ii), the cell membrane fractions may be obtained by differential centrifugation. The differential centrifugation may be performed twice by primary centrifugation and secondary centrifugation. The primary centrifugation may be performed at a speed of 8,000×g to 11,000xg. Specifically, the primary centrifugation may be performed at a speed of8,000×g to 11,000×g, 8,500×g to 10,500×g, or9,000×g to 10,000×g. In an embodiment of the present invention, the primary centrifugation was performed under the conditions of 9,500×g, 5 minutes, and 4 °C. The secondary centrifugation may be performed at a speed of 140,000×g to 160,000×g. Specifically, the secondary centrifugation may be performed at a speed of 140,000×g to 160,000×g, 141,000×g to 159,000×g, 142,000×g to 158,000×g, 143,000×g to 157,000×g, 144,000×g to 156,000×g, 145,000×g to 155,000×g, 146,000×g to 154,000×g, 147,000×g to 153,000×g, 148,000×g to 152,000×g, or 149,000×g to 151,000×g. In an embodiment of the present invention, the second centrifugation was performed under the conditions of 150,000×g, 90 minutes, and 4 °C.

In step iii), the microfluidizer may be repeatedly performed three times to seven times at a pressure of 15,000 psi to 25,000 psi. Specifically, in step iii), the microfluidizer may be performed at a pressure of 15,000 psi to 25,000 psi, 16,000 psi to 24,000 psi, 17,000 psi to 23,000 psi, 18,000 psi to 22,000 psi, or 19,000 psi to 21,000 psi. In addition, in step iii), the microfluidizer may be repeatedly performed three times to seven times or four times to six times. In one embodiment of the present invention, in step iii), the microfluidizer was repeatedly performed five times at a pressure of 20,000 psi.

In step iii), an extraction buffer containing a surfactant may be added to the cell membrane fractions. In step iii), when there is no surfactant, the membrane fractions may only be disrupted again, and the membrane proteins may not be extracted. In this case, the role of the microfluidizer is to disrupt the membrane fractions very small and homogeneous in the size of nanoparticles so that the surfactant can efficiently solubilize membranes and extract membrane proteins.

Specifically, the membrane proteins have hydrophobic properties, and thus are not dissolved in an aqueous solution, but are embedded in the membrane that is also hydrophobic. The membrane fractions are in a state in which the membranes and the membrane proteins agglomerate as a lump, and in this state, even when a surfactant is treated, only the surface of the membrane fraction lump is solubilized, and thus efficient extraction may not be performed. If the microfluidizer is used, the membranes may be disrupted finely and evenly into nano-sized pieces, and the surface area, to which the surfactant may act, increases, so that the extraction may be efficiently performed.

The surfactant may be any one selected from the group consisting of SDS, TritonX-100, Nonidet P-40, octyl-b-D-glucopyranoside, n-dodecyl-b-D-maltoside, and Zwittergent 3-16. Specifically, the lysis buffer containing the surfactant may include Tris, TritonX-100, NaCl, EDTA, and a protease inhibitor. In an embodiment of the present invention, a solution containing 25 mM Tris, 1% TritonX-100, 40 mM NaCl, 1 mM EDTA, and a protease inhibitor, and having pH 8.5 was used as a lysis buffer containing a surfactant during the disruption of the cell membrane fractions.

In step iv), ultra-centrifugation may be used to separate the target proteins. The ultra-centrifugation may be performed at a speed of 80,000×g to 120,000×g for 30 minutes to 90 minutes. Specifically, the ultra-centrifugation may be performed at a speed of 80,000×g to 120,000×g, 85,000×g to 115,000×g, 90,000×g to 110,000×g, or 95,000×g to 105,000×g for 30 minutes, 40 minutes, 50 minutes, 60 minutes, 70 minutes, 80 minutes, or 90 minutes. In an embodiment of the present invention, the ultra-centrifugation was performed under the conditions of 100,000×g, 40 minutes, and 4 °C.

The target proteins may be membrane proteins. The membrane proteins may be spike proteins of a virus. Specifically, the membrane proteins may be spike proteins of the Middle East respiratory syndrome coronavirus. The spike proteins of the Middle East respiratory syndrome coronavirus (MERS-CoV S) is also referred to as an S protein, and means a surface glycoprotein of the Middle East respiratory syndrome coronavirus. In addition, the spike protein is a glycoprotein expressed as a trimer constituting a spike or a peplomer on the surface of the coated Middle East respiratory syndrome coronavirus particle.

The cells may be animal cells. Specifically, the animal cells may be insect cells, and the insect cells may be any one selected from the group consisting of BT1-Tn-SB1-4 cells of *Trichoplusiani*, LD652Y cells of *Lymantria dispar,* Sf9 cells of *Spodoptera frugiperda,* Sf21 cells of *Spodoptera frugiperda*, Kc1 cells of *Drosophila,* SL2 cells of *Drosophila,* and mosquito cell lines. Preferably, the insect cells may be the Sf9 cells of *Spodoptera frugiperda.* In an embodiment of the present invention, the Sf9 cells of *Spodoptera frugiperda* were used.

### Method for Purifying Target Proteins by Using Homogenizer

The target protein purification method may include: in step i), performing homogenization 10 times to 20 times; in step ii), performing primary centrifugation at a speed of 2,500×g to 3,500×g for 10 minutes to 30 minutes and then performing secondary centrifugation at a speed of 4,000×g to 6,000×g for 20 minutes to 40 minutes; in step iii), performing homogenization three times to seven times; and in step iv), performing centrifugation at a speed of 4,000×g to 6,000×g for 20 minutes to 40 minutes.

In step i), the homogenization may be performed 10 times to 20 times by using a homogenizer. Specifically, in step i), the homogenization may be performed 10 times, 11 times, 12 times, 13 times, 14 times, 15 times, 16 times, 17 times, 18 times, 19 times, or 20 times by using a homogenizer. In one embodiment of the present invention, the homogenization was performed 12 times.

In step i), a lysis buffer that does not contain a surfactant may be used during cell disruption. Specifically, the lysis buffer that does not contain the surfactant may include Tris, EDTA, NaCl, and a protease inhibitor. In one embodiment of the present invention, a solution including 25 mM Tris, 1 mM EDTA, 100 mM NaCl and a protease inhibitor and having pH 8.5 was used as a lysis buffer without containing a surfactant during the cell disruption.

In step ii), the cell membrane fractions may be obtained by differential centrifugation. In step ii), the cell membrane fractions may be obtained by performing the primary centrifugation at a speed of 2,500×g to 3,500×g for 10 minutes to 30 minutes and then performing the secondary centrifugation at a speed of 4,000×g to 6,000×g for 20 minutes to 40 minutes. The primary centrifugation may be performed at a speed of 2,500×g to 3,500×g, 2,600×g to 3,400×g, 2,700×g to 3,300×g, 2,800×g to 3,200×g, or 2,900×g to 3,100×g for 10 minutes, 15 minutes, 20 minutes, 25 minutes, or 30 minutes. In an embodiment of the present invention, the primary centrifugation was performed at a speed of 3,000×g for 20 minutes. The secondary centrifugation may be performed at a speed of 4,000×g to 6,000×g, 4,400×g to 5,900×g, 4,800×g to 5,800×g, or 5,200×g to 5,700×g for 20 minutes, 25 minutes, 30 minutes, 35 minutes, or 40 minutes. In an embodiment of the present invention, the secondary centrifugation was performed at a speed of 5,400×g for 30 minutes.

In step iii), the homogenization may be performed three to seven times by using a homogenizer. Specifically, in step iii), the homogenization may be performed three times, four times, five times, six times, or seven times by using a homogenizer. In one embodiment of the present invention, the homogenization was performed five times.

In step iii), an extraction buffer containing a surfactant may be added to the cell membrane fractions. The surfactant may be any one selected from the group consisting of SDS, TritonX-100, Nonidet P-40, octyl-b-D-glucopyranoside, n-dodecyl-b-D-maltoside, and Zwittergent 3-16. Specifically, the lysis buffer containing the surfactant may include Tris, TritonX-100, NaCl, and EDTA. In an embodiment of the present invention, a solution containing 0.5% triton100, 130 mM NaCl, 25 mM Tris, and 1 mM EDTA was used as a lysis buffer containing a surfactant during the disruption of the cell membrane fractions.

In step iv), the centrifugation may be performed at a speed of 4,000×g to 6,000×g for 20 minutes to 40 minutes. The centrifugation may be performed at a speed of 4,000×g to 6,000×g, 4,400×g to 5,900×g, 4,800×g to 5,800×g, or 5,200×g to 5,700×g for 20 minutes, 25 minutes, 30 minutes, 35 minutes, or 40 minutes. In an embodiment of the present invention, the centrifugation was performed at a speed of 5,400×g for 30 minutes.

The target proteins are the same as described in the Method for Purifying Target Proteins by Using Microfluidizer.

### Method for Purifying Target Proteins by Using Sonicator

The target protein purification method may include: in step i), performing sonication for 5 minutes to 30 minutes; in step ii), performing primary centrifugation at a speed of 100×g to 1,000×g for 1 minute to 3 minutes and then performing secondary centrifugation at a speed of 12,000×g to 14,000×g for 10 minutes to 20 minutes; in step iii), performing sonication for 10 minutes to 30 minutes; and in step iv), performing centrifugation at a speed of 10,000×g to 15,000×g for 20 minutes to 40 minutes.

In step i), the sonication may be performed for 5 minutes to 30 minutes by a sonicator. Specifically, in step i), the sonication may be performed for 5 minutes, 10 minutes, 15 minutes, 20 minutes, 25 minutes or 30 minutes by using a sonicator. In an embodiment of the present invention, the sonication was performed for 20 minutes.

In step i), a hypotonic lysis buffer may be used for the sonication. Specifically, the hypotonic lysis buffer may include Tris buffer. In one embodiment of the present invention, a solution containing 10 mM Tris buffer and having pH 7.5 was used as a hypotonic lysis buffer during the cell disruption. In this case, during the cell disruption in step i), a surfactant may be additionally treated.

In step ii), the cell membrane fractions may be obtained by differential centrifugation. In step ii), the cell membrane fractions may be obtained by performing the primary centrifugation at a speed of 100×g to 1,000×g for 1 minutes to 3 minutes and then performing the secondary centrifugation at a speed of 12,000×g to 14,000×g for 10 minutes to 20 minutes. The primary centrifugation may be performed at a speed of 100×g to 1,000×g, 200×g to 900×g, 300×g to 800×g, or 400×g to 700×g for 1 minute, 2 minutes, or 3 minutes. In an embodiment of the present invention, the primary centrifugation was performed at a speed of 500×g for 1 minute. The secondary centrifugation may be performed at a speed of 12,000×g to 14,000×g, 12,100×g to 13,900×g, 12,200×g to 13,800×g, 12,300×g to 13,700×g, 12,400×g to 13,600×g, 12,500×g to 13,500×g, 12,600×g to 13,400×g, 12,700×g to 13,300×g, 12,800×g to 13,200×g, or 12,900×g to 13,100×g for 10 minutes, 15 minutes, or 20 minutes. In an embodiment of the present invention, the secondary centrifugation was performed at a speed of 13,000×g for 15 minutes.

In step iii), the sonication may be performed for 10 minutes to 30 minutes. Specifically, in step iii), the sonication may be performed for 10 minutes, 15 minutes, 20 minutes, 25 minutes, or 30 minutes by using a sonicator. In an embodiment of the present invention, the sonication was performed for 20 minutes.

In step iii), an extraction buffer containing a surfactant may be added to the cell membrane fractions. The surfactant may be any one selected from the group consisting of SDS, TritonX-100, Nonidet P-40, octyl-b-D-glucopyranoside, n-dodecyl-b-D-maltoside, and Zwittergent 3-16. Specifically, the lysis buffer containing the surfactant may include Tris, TritonX-100, NaCl, and EDTA. In an embodiment of the present invention, a solution containing 40 mM Tris, 30 mM NaCl, 1 mM EDTA, 1% Triton X-100, and having pH 7.8 was used as a lysis buffer containing a surfactant during the disruption of the cell membrane fractions.

In step iv), the centrifugation may be performed at a speed of 10,000×g to 15,000×g for 20 minutes to 40 minutes. The centrifugation may be performed at a speed of 10,000×g to 15,000×g, 10,500×g to 14,500×g, 11,000×g to 14,000×g, 11,500×g to 13,800×g, 12,000×g to 13,600×g, or 12,500×g to 13,400×g for 20 minutes, 25 minutes, 30 minutes, 35 minutes, or 40 minutes. In an embodiment of the present invention, the centrifugation was performed at a speed of 13,000×g for 30 minutes.

The target proteins are the same as described in the Method for Purifying Target Proteins by Using Microfluidizer.

### Modes for the Invention

Hereinafter, the present invention will be described in more detail with reference to the following examples. However, the following examples are only for illustrating the present invention, and the scope of the present invention is not limited thereto.

### I. Optimization of Disruption and Elution Conditions in Process of Separating and Purifying Membrane Proteins

Sf9 cells were cultured at a concentration of 1×10⁶ cells/mL, and then was infected with MERS-CoV S recombinant baculovirus with an MOI of 0.1, and harvested after 3-5 days. The harvested Sf9 cells expressed the MERS-CoV S proteins on the surface and on the endoplasmic reticulum (ER) membrane. In order to exclude impurities from the harvested Sf9 cells as much as possible, and to elute the S proteins expressed in the cell membranes, cell disruption, centrifugation, and S protein elution conditions were studied.

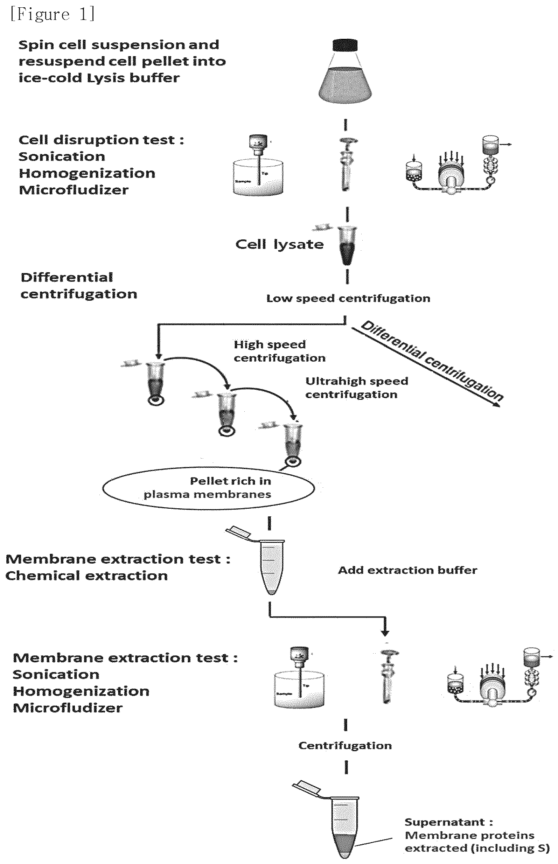

In this case, homogenization, sonication, and microfluidizer technologies were compared and tested in order to find an optimal cell disruption method. In order to remove impurities from the disrupted cells and obtain only plasma membranes and ER membranes, a centrifugation condition experiment was performed. In addition, in order to elute the S proteins from the cell membranes, the homogenization, sonication, and microfluidizer technologies were compared and tested (FIG. 1).

### Example 1. Sf9 Cell Preparation and Recombinant MERS-CoV S Baculovirus Inoculation

### Example 1.1. Sf9 Cell Culture

In order to prepare Sf9 cells for use in the experiment, Sf9 cells were thawed, first. Specifically, one vial of a Sf9 cell stock was taken out of liquid nitrogen and thawed in a constant-temperature water bath at 28 °C for 2 minutes. When the Sf9 cells were almost thawed, the surface was wiped with 70% ethanol and put into a biosafety cabinet (BSC). Then, the Sf9 cells were put into a 15-mL falcon tube containing 9.0 mL of medium at room temperature.

Centrifugation was performed at 125×g for 5 minutes. The supernatant was removed, cell pellets were resuspended in 10 mL of medium, and then put into a 25-cm² flask. The flask was placed in an incubator at 27 °C and incubated for 48 hours. After 48 hours, the culture medium was removed and a fresh 10-mL medium was added thereto. Again, the flask was placed in the incubator at 27 °C and further incubated for 48 hours.

After 48 hours, the Sf9 cells attached to the bottom of the flask were detached by using a pipette aid and transferred to a 75-cm² flask containing 15 mL of medium. Thereafter, the flask was placed in the incubator at 27 °C and further incubated for 72 hours. After 72 hours, the Sf9 cells attached to the bottom of the flask were detached by using the pipette aid and transferred to a conical tube containing 50 mL of medium.

A vial cell density was calculated by using a hemacytometer. A 50 mL culture medium was prepared so that the initial cell concentration was 5.0×10⁵ cells/mL, and then transferred to a 125-mL spinner flask. The spinner flask was incubated in a shaking incubator at 27 °C for 48 hours at 90 rpm. After 48 hours, cell viability and cell concentration were checked. In this case, when the cell concentration reached 2.0×10⁶ to 3.0×10⁶ cells/mL, the cells were transferred to a fresh medium so that the initial cell concentration reached 5.0×10⁵ cells/mL. In the same manner, the medium was replaced with a fresh medium once every 2-3 days.

### Example 1.2. Recombinant MERS-CoV S Baculovirus Inoculation and Acquisition

The Sf9 cells of Example 1.1 were seeded in a 1-L spinner flask at a concentration of 1×10⁶ cells/mL. Thereafter, the recombinant MERS-CoV S *Baculovirus* RWVSS was taken out and thawed in a constant-temperature water bath at 27 °C for 2 minutes. Fully thawed recombinant MERS-CoV S *Baculovirus* RWVSS was inoculated into the Sf9 cells with an MOI of 0.1. After 3-5 days of the inoculation, the Sf9 cells infected with the recombinant MERS-CoV S *Baculovirus* RWVSS were harvested together with the medium. Then, the harvested medium was centrifuged at 4 °C for 15 minutes at 6,000×g and precipitated.

### Example 2. Disruption and Elution Experiments of MERS-CoV S Proteins

### Example 2.1. Experiment with Homogenizer

The Sf9 cells harvested in Example 1.2 were incubated for 30 minutes with a cold lysis buffer (25 mM Tris, 1 mM EDTA, 100 mM NaCl, pH 8.5, and a protease inhibitor) added, cooled on ice, and then stroked 12 times on ice by using a homogenizer. Thereafter, centrifugation was performed for 20 minutes at 4 °C and 3,000×g. Then, the pellets were removed, only the supernatant was separated, and centrifuged again at 4 °C and 5,400×g for 30 minutes. After the supernatant was removed and pellets were collected and treated with a cooled buffer containing 0.5% Triton100, 130 mM NaCl, 25 mM Tris, and 1 mM EDTA, followed by five times of strokes with a homogenizer. Then, the extract was centrifuged at 4 ° C and 5,400×g for 30 minutes to precipitate an insoluble fraction, and a supernatant was taken therefrom to obtain a soluble membrane fraction.

Samples were prepared by sampling the obtained fraction. The samples were taken out by 14 µL each and then placed in an EP tube. Thereafter, 5 µL of 4X SDS loading buffer and 1 µL of 2-mercaptoethanol were added to the EP tube. After shaking the EP tube, the EP tube was allowed to stand at 96 °C for 8 minutes. After 8 minutes, the EP tube was spun down for 1 minute at 6,000 rpm.

Then, the samples that had been pre-treated were loaded by 15 µL each on a 4% to 12% gradient PAGE gel for 40 minutes at 200 volts. At this time, 8 µL of pre-stained protein marker was loaded together. In this case, the samples loaded for each lane are shown in Table 1 below.

**[Table 1]**

| Lane | Samples |
|---|---|
| 1 | Whole cell |
| 2 | 3,000×g 20 min, supernatant |
| 3 | 3,000×g 20 min, sup at 5,400×g, 30 min, supernatant |
| 4 | 3,000×g; 20 min, pellets |
| 5 | 3,000×g, 20 min, pellets, subjected to triton extraction, centrifuged at 5,400×g, 30 min, supernatant (soluble extract) |
| 6 | 3,000×g, 20 min, pellets, subjected to triton extraction, centrifuged at 5,400×g, 30 min, supernatant |

Then, the gel was separated from the electrophoresis device, and the gel was carefully removed from the plate, and unnecessary parts were cut out. A transfer kit (Thermo Fisher, iBlot 2 Transfer Stacks, PVDF, regular size (Cat. No. IB24001)) was prepared, and then used to transfer the gel to a membrane.

Then, 20 mL of a 5% skim milk solution was added to a western blot container, the membrane was added thereto, and the membrane was shaken at 20 rpm, and a blocking process was performed at 4 °C for 4 hours or more.

15 mL of a 0.05% skim milk solution was added to a 15-mL falcon tube, and primary antibodies were added at a ratio of 1:2,000 to prepare a primary antibody (Thermofisher # PA5-81786)-attached solution, wherein the primary antibody is a polyclonal antibody that specifically binds to a MERS CoV spike protein.

The membrane after the blocking process had been completed was transferred to a western blot container, 15 mL of the primary antibody-attached solution was added thereto, and the membrane was reacted at 4 °C for at least 1 hour by using a shaker at 20 rpm.

The membrane was washed three times with 1X PBS-T buffer. 15 mL of a 1X PBS-T buffer was added to a 15-mL falcon tube, and secondary antibodies which is an HRP-labeled Goat anti-rabbit IgG (Abeam # ab205718), were added at a ratio of 1:5,000 to prepare a secondary antibody-attached solution. The washed membrane was transferred to a western blot container, and 15 mL of a previously made secondary antibody solution was added thereto and shaken at 20 rpm for 1 hour. Then, the membrane was washed three times with 1X PBS-T buffer.

In the final washing step, a cassette, a transparent plastic bag, and an ECL kit (Thermo Fisher #32134) stored at 4 °C were performed according to the manufacturer's manual, the cassette cover was closed to shield light, and the result was confirmed by exposing the cassette using an X-ray film in a dark room.

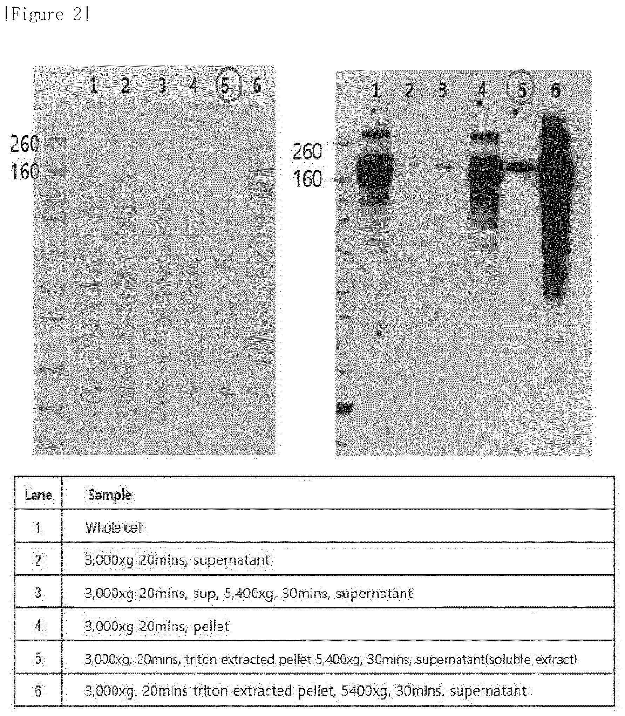

As a result, the proteins were extracted as a soluble membrane fraction, but a small amount of the MERS-CoV S proteins were extracted. Most of the MERS-CoV S proteins were not dissolved and precipitated as pellets (FIG. 2).

### Example 2.2. Experiment with Sonicator

The Sf9 cells harvested in Example 1.2 were resuspended in a cold hypotonic lysis buffer. Thereafter, the disruption experiments were carried out under six conditions listed in Table 2 below.

**[Table 2]**

| **Conditions (all performed at 4 °C)** | |
|---|---|
| 1 | hypotonic lysis buffer |
| 2 | hypotonic lysis buffer + sonication (5min) |
| 3 | hypotonic lysis buffer + sonication (20min) |
| 4 | hypotonic lysis buffer + 0.05% TritonX-100 |
| 5 | hypotonic lysis buffer + 0.05% TritonX-100 + sonication (5min) |
| 6 | hypotonic lysis buffer + 0.05% TritonX-100 + sonication (20min) |

After the disruption experiments under six conditions were performed, whether the Sf9 cells were disrupted was confirmed by a microscope, and the results are shown in Table 3 below.

**[Table 3]**

| **Disruption conditions** | **Clearly visible cells** | **Blurred visible cells** |
|---|---|---|
| ① hypotonic lysis buffer | 280 | 216 |
| ② hypotonic lysis buffer + sonication (5 min) | 163 | 146 |
| ③ hypotonic lysis buffer + sonication (20 min) | 30 | 7 |
| ④ hypotonic lysis buffer + 0.05% TritonX-100 | 185 | 174 |
| ⑤ hypotonic lysis buffer + 0.05% TritonX-100 + sonication (5 min) | 206 | 182 |
| ⑥ hypotonic lysis buffer + 0.05% TritonX-100 + sonication (20 min) | 69 | 75 |

As shown in Table 3, when the hypotonic lysis buffer was added and sonicated for 20 minutes, the disruption was performed best. The samples under the conditions of No. 3 and No. 6, which had been most dissolved, were centrifuged under two conditions shown in Table 4.

**[Table 4]**

| **Sink conditions (4 °C)** | |
|---|---|
| 1 | 500×g, 1 min (nuclei removal) → 13,000×g, 15 min (membrane fraction) |
| 2 | 13,000×g, 15 min (membrane fraction without nuclei removal) |

Samples fractionated under two conditions were subjected to the addition of an extraction buffer (40 mM Tris, 30 mM NaCl, 1 mM EDTA, 1% Trixon X-100, pH 7.8), followed by sonication for 20 minutes and elution. Then, the extract was centrifuged at 13,000×g for 30 minutes. Samples were prepared by sampling the centrifuged solution. Thereafter, western blotting was performed in the same manner as in Example 2.1. The conditions of the extraction process are shown in Table 5 below.

**[Table 5]**

| Disrupted samples | Differential centrifugation | | After extraction |
|---|---|---|---|
| | 500×g, 1 minute | Supernatant 13,000×g, 15 min | 13,000×g, 30 minute |
| (3) hypotonic lysis buffer + sonication (20 min) | pellet: sample 3 nuclei | supernatant (sup): cytosol, sample 5 | supernatant: soluble S, sample 13 |
| | | pellet: sample 9, extraction | pellet: sample 17 |
| | not performed | supernatant (sup): cytosol, sample 6 | supernatant: soluble, sample 14 |
| | | pellet: sample 10, extraction | pellet: sample 18 |
| ⑥ hypotonic lysis buffer + 0.05% TritonX-100 + sonication (20 min) | pellet: sample 4 nuclei | supernatant (sup): cytosol, sample 7 | supernatant: soluble S, sample 15 |
| | | pellet: sample 11, extraction | pellet: sample 19 |
| | not performed | supernatant (sup): cytosol, sample 8 | supernatant: soluble, sample 16 |
| | | pellet: sample 12, extraction | pellet: sample 20 |

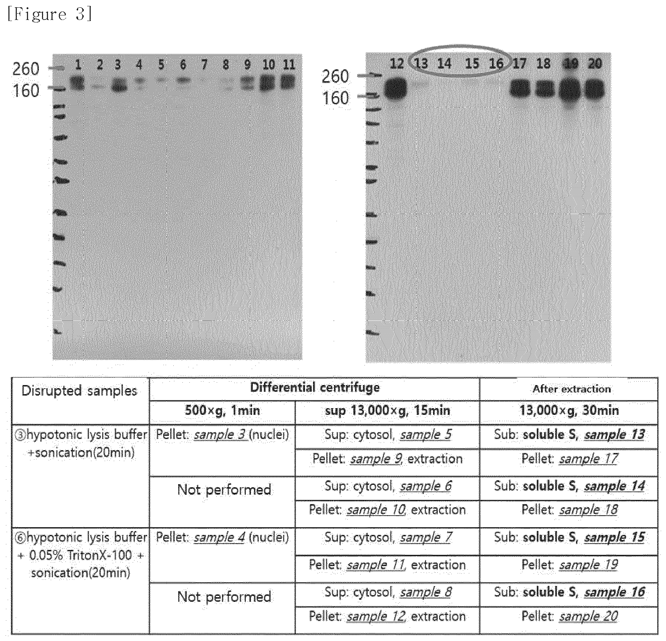

As a result, only a portion of the MERS-CoV S proteins was dissolved and eluted even with 1% Triton X-100 and 20 min sonication, and most of them were precipitated as pellets. In addition, among two bands around 160 kDa, only the upper band was dissolved and eluted, and the lower band was not eluted at all. The upper band is estimated to be a protein which is normally moved to the membrane after a post-translational modification such as glycosylation, and the lower band is estimated to be a protein which is not properly subjected to the post-translational modification due to the overexpression and protein folding errors and is not dissolved and forms an aggregate (FIG. 3).

In addition, the concentration of TritonX-100 has already much exceeded the CMC value, and since the concentration has reached the maximum concentration generally used for protein elution, further increase is not expected to be effective, and an increase in reaction temperature or time was not attempted because there are high risks of decreasing stability of the target protein, the MERS-CoV S protein, and increasing protein degradation by proteases. Instead, additional experiments were conducted by using a microfluidizer to further facilitate elution by increasing physical force.

### Example 2.3. Experiment with Microfluidizer

The Sf9 cells harvested in Example 1.2 were resuspended with a lysis buffer (25 mM Tris, 1 mM EDTA, 100 mM NaCl, a protease inhibitor, and pH 8.5) stored at 4 °C. Then, cell suspension was disrupted three times to five times by using a microfluidizer at 5,000 psi.

Cell lysates were centrifuged at 4 °C and 9,500×g for 15 minutes to remove nuclei, mitochondria, lysosome, and peroxisome. Then, the supernatant was ultra-centrifuged by using an ultra-centrifuge at 4 °C and 150,000×g for 90 minutes to remove cytosol or the like to obtain pellets (plasma membrane, ER, and Golgi).

The obtained pellets were resuspended in an extraction buffer stored at 4 °C (25 mM Tris, 1% TritonX-100, 40 mM NaCl, 1 mM EDTA, a protease inhibitor, and pH 8.5). The membrane pellets were disrupted three times to five times by using a microfluidizer at 20,000 psi. The disrupted sample was ultra-centrifuged by using an ultra-centrifuge at 4 °C and 100,000×g for 40 minutes to obtain a supernatant.

Samples were prepared by sampling the supernatant. Thereafter, western blotting was performed in the same manner as in Example 2.1. In this case, the samples loaded for each lane are shown in Table 6 below.

**[Table 6]**

| Lane | Samples |
|---|---|
| 1 | Lysis |
| 2 | 4,500×g sup |
| 3 | 150,000×g pellet |
| 4 | extraction |
| 5 | 10,000×g sup |

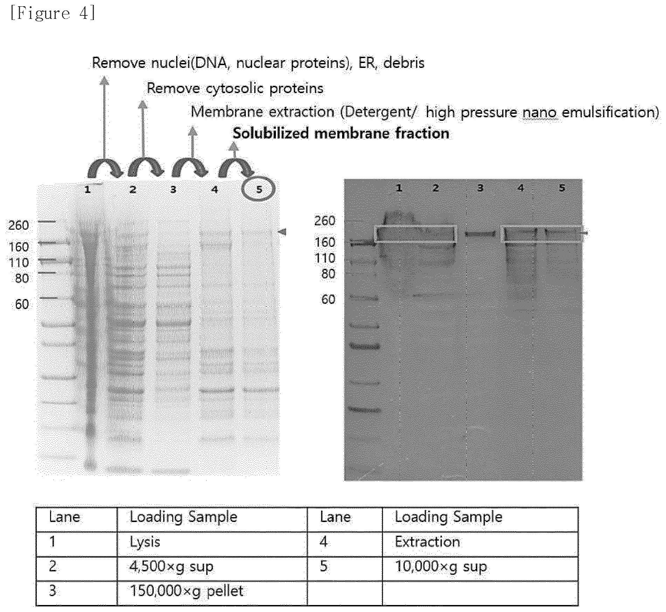

As a result, it was confirmed that nuclei, DNA, endoplasmic reticulum, cytosolic proteins, and the like were removed through the cell disruption by using a microfluidizer and differential centrifugation, and the cell membrane fractions containing the MERS-CoV S proteins were eluted properly. In addition, it was confirmed that the elution of the soluble MERS-CoV S proteins was also performed very properly compared to using other methods. In addition, as shown in FIG. 4, in the case of the result of western blotting at the right side, there was a phenomenon in which the film was burned out due to a large amount of the target proteins, and the colored part was whitened (arrow parts of lanes 1, 2, 4, and 5 in FIG. 4). Accordingly, it was confirmed that the disruption and elution experiments using the microfluidizer may be usefully applied to a subunit vaccine requiring the expression of a target membrane protein in insect cells.

### Example 3. Optimization of Disruption and Elution Conditions in Process of Separating and Purifying Membrane Proteins

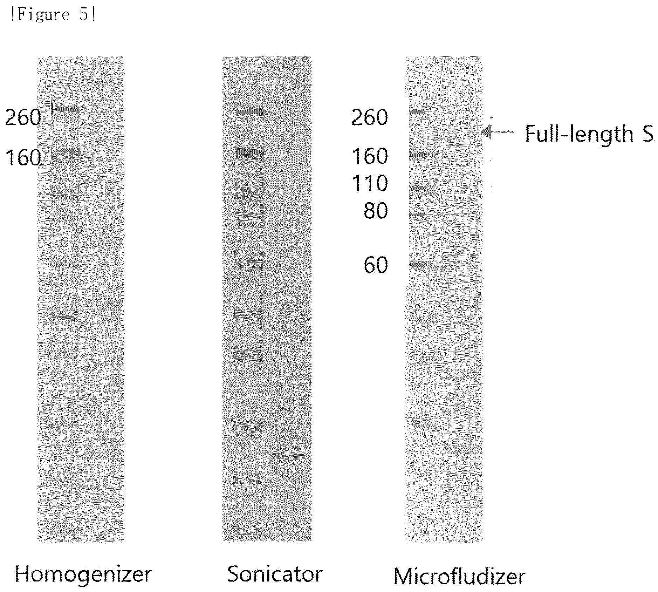

It was confirmed that when the cells were disrupted and eluted by using the homogenizer of Example 2.1 or the sonicator of Example 2.2, the final protein yield after the completion of purification was at most 0.1 mg/L, but when the microfluidizer of Example 2.3 was used, the final protein yield was increased to about 10 mg/L by at least 100 times. Through this, it is confirmed that the method of cell disruption and elution by using a microfluidizer is most suitable (FIG. 5).

In particular, when cells are disrupted and eluted by using a microfluidizer, it is advantageous to remove most foreign substances (cell lysates, cytosolic proteins, nuclei, mitochondria, peroxisomes, lysosomes, and the like) except plasma membranes and membrane-associated proteins by applying cell fractionation by using differential centrifugation, thereby improving purity, and particularly, it is confirmed that DNA contamination and protein damage by proteases can be reduced by removing nuclei, peroxisomes, and lysosomes.

## Claims

1. A method for purifying target proteins, the method comprising the steps of:
i) disrupting cells using a sonicator, a homogenizer, or a microfluidizer;
ii) obtaining cell membrane fractions;
iii) disrupting the cell membrane fractions using the sonicator, the homogenizer, or the microfluidizer; and
iv) separating the target proteins.

2. The method of claim 1, wherein in step i), the microfluidizer is repeatedly performed once to seven times at a pressure of 3,500 psi to 6,000 psi.

3. The method of claim 1, wherein in step ii), the cell membrane fractions are obtained by differential centrifugation.

4. The method of claim 3, wherein the differential centrifugation is performed twice by primary centrifugation and secondary centrifugation.

5. The method of claim 4, wherein the primary centrifugation is performed at a speed of 8,000×g to 11,000×g.

6. The method of claim 4, wherein the secondary centrifugation is performed at a speed of 140,000×g to 160,000×g.

7. The method of claim 1, wherein in step iii), the microfluidizer is repeatedly performed three times to seven times at a pressure of 15,000 psi to 25,000 psi.

8. The method of claim 1, wherein in step iii), an extraction buffer containing a surfactant is added to the cell membrane fractions.

9. The method of claim 8, wherein the surfactant is any one selected from the group consisting of SDS, TritonX-100, Nonidet P-40, octyl-b-D-glucopyranoside, n-dodecyl-b-D-maltoside, and Zwittergent 3-16.

10. The method of claim 1, wherein in step iv), the separating uses ultra-centrifugation.

11. The method of claim 10, wherein the ultra-centrifugation is performed at a speed of 80,000×g to 120,000×g.

12. The method of claim 1, wherein the target proteins are membrane proteins.

13. The method of claim 1, wherein the cells are animal cells.

14. The method of claim 13, wherein the animal cells are insect cells.

15. The method of claim 14, wherein the insect cells are any one selected from the group consisting of BT1-Tn-SB1-4 cells of *Trichophisiani*, LD652Y cells of *Lyman tria dispar*, Sf9 cells of *Spodoptera frugiperda*, Sf21 cells of *Spodoptera frugiperda*, Kc1 cells of *Drosophila,* SL2 cells of *Drosophila,* and mosquito cell lines.

16. The method of claim 1, wherein the target protein purification method comprises: in step i), performing homogenization 10 times to 20 times; in step ii), performing primary centrifugation at a speed of 2,500×g to 3,500×g for 10 minutes to 30 minutes and then performing secondary centrifugation at a speed of 4,000×g to 6,000×g for 20 minutes to 40 minutes; in step iii), performing homogenization three times to seven times; and in step iv), performing centrifugation at a speed of 4,000×g to 6,000×g for 20 minutes to 40 minutes.

17. The method of claim 1, wherein the target protein purification method comprises: in step i), performing sonication for 5 minutes to 30 minutes; in step ii), performing primary centrifugation at a speed of 100×g to 1,000×g for 1 minute to 3 minutes and then performing secondary centrifugation at a speed of 12,000×g to 14,000×g for 10 minutes to 20 minutes; in step iii), performing sonication for 10 minutes to 30 minutes; and in step iv), performing centrifugation at a speed of 10,000×g to 15,000×g for 20 minutes to 40 minutes.

18. The method of claim 17, wherein in step i), a surfactant is additionally treated.
